# EUROPÄISCHE PATENTSCHRIFT

(11) **EP 2 367 495 B1**
(45) Veröffentlichungstag und Bekanntmachung des Hinweises auf die Patenterteilung: **11.09.2013**
(21) Anmeldenummer: 09781246.5
(22) Anmeldetag: 29.07.2009
(51) Int. Cl.: A61B 18/24

(54) **LASERAPPLIKATOR**
LASER APPLICATOR
APPLICATEUR LASER

(30) Priorität: 20.11.2008 DE 102008058148
(43) Veröffentlichungstag der Anmeldung: 28.09.2011
(73) Patentinhaber: Vimecon GmbH, 52134 Herzogenrath (DE)
(72) Erfinder: MARKUS, Kai, Ulf, 52249 Eschweiler (DE)
(74) Vertreter: von Kreisler Selting Werner
(86) Internationale Anmeldenummer: PCT/EP2009/059818
(87) Internationale Veröffentlichungsnummer: WO 2010/057689

(56) Entgegenhaltungen:
- WO-A-2004/078045
- WO-A-2005/120379
- WO-A-2007/035456
- US-A- 5 025 778
- US-A1- 2003 199 736

## Beschreibung

Die Erfindung betrifft einen Laserapplikator mit einem langgestreckten Katheter, der mindestens ein umfangsmäßig geschlossenes Lumen enthält, und einem längs des Katheters verlaufenden Lichtleiter, der in einem distalen Endabschnitt des Katheters einen Auskoppelbereich aufweist.

Ein derartiger Laserapplikator ist beschrieben in WO 2007/118745 A1 (Vimecon), deren Inhalt durch Verweis in die vorliegende Beschreibung aufgenommen wird. Der bekannte Laserapplikator weist einen langgestreckten flexiblen Katheter auf, der einen Lichtleiter enthält. Der distale Endabschnitt ist zu einer lasso-ähnlichen Schleife geformt, deren Ebene quer zu dem Hauptteil des Katheters verläuft. Am proximalen Ende wird Laserstrahlung in den Lichtleiter eingespeist. Am distalen Ende des Katheters befindet sich ein Auskoppelbereich, an dem die Energie seitlich aus dem Lichtleiter ausgekoppelt wird und den Katheter verlässt.

Der Laserapplikator dient insbesondere zur Behandlung von Vorhofflimmern und anderen Herz-Rhythmus-Störungen. Mit ihm kann Herzgewebe durch Umwandlung von Lichtenergie in thermische Energie verödet werden. Durch die aus dem Lichtleiter austretende Laserstrahlung wird das umgebende Gewebe auf Werte von über 60 °C erhitzt, was zu einer Denaturierung von Proteinen und zum Ausbilden einer elektrisch inaktiven Narbe führt.

In DE 10 2006 039 471 B3 ist ein Laserapplikator beschrieben, der einen Katheter mit einem Lichtleiter enthält. In einem distalen Endabschnitt des Katheters hat der Mantel des Lichtleiters eine Aussparung an der Licht seitlich aus dem Lichtleiter austritt. Während der intakte Mantel des Lichtleiters für Totalreflexion sorgt, wodurch die Lichtenergie in Längsrichtung des Lichtleiters transportiert wird, bewirken die Aussparungen an der Grenze des Kernes des Lichtleiters eine Brechung, wodurch Strahlungsenergie ausgekoppelt wird.

Ein Laserapplikator nach dem Oberbegriff des Patentanspruchs 1 ist beschrieben in WO 2007/035456 A1. Der Laserapplikator hat einen langgestreckten Katheter mit mehreren Lumen. In einer Nut, die längs des Katheters verläuft, ist ein Führungsschlitten verschiebbar angeordnet. An diesem ist der Lichtleiter befestigt. Der Führungsschieber hat ein Lichtaustrittsfenster, aus dem der endseitig aus dem Lichtleiter austretende Lichtstrahl seitlich aus dem Katheter herausgeführt wird.

In WO 2004/078045 A1 ist ein Laserapplikator beschrieben, der zahlreiche Lichtleitfasern enthält, die jeweils in äußeren Nuten eines Trägerkörpers angeordnet sind. Das Licht tritt endseitig aus den Lichtleitfasern aus und wird von einer Schrägfläche des Trägerkörpers radial nach außen reflektiert.

Die vorliegende Erfindung befasst sich mit der konstruktiven Gestaltung eines Laserapplikators. Ihr liegt die Aufgabe zugrunde, einen Laserapplikator zu schaffen, der einfach und kostengünstig herstellbar ist und sich damit besonders für eine industrielle Produktion eignet.

Der erfindungsgemäße Laserapplikator ist durch den Patentanspruch 1 definiert.

Erfindungsgemäß ist mindestens der Auskoppelbereich des Lichtleiters in eine seitlich offene Nut des Katheters eingesetzt. Dadurch besteht die Möglichkeit, den Lichtleiter vor seinem Einsetzen in den Katheter zu bearbeiten, insbesondere im Auskoppelbereich den Mantel (cladding) des Lichtleiters teilweise zu entfernen. Der so bearbeitete Lichtleiter kann dann seitlich in der längslaufenden Nut des Katheters platziert werden. Die Fixierung erfolgt anschließend mit einem lichtdurchlässigen Material, insbesondere einem entsprechenden Kleber. Die Anbringung des Lichtleiters in einer äußeren Nut des Katheters bewirkt eine erhebliche Vereinfachung des Herstellungsverfahrens des Laserapplikators.

Generell kann die Nut an der Außenseite des Katheters jeden beliebigen Querschnitt haben, also beispielsweise rechteckig oder halbkreisförmig sein. Gemäß einer bevorzugten Ausgestaltung der Erfindung ist die Nut V-förmig und sie weist Flanken auf, die mit einer Reflexionsschicht versehen sind. Strahlungsenergie, die aus dem Lichtleiter seitlich austritt und in dem umgebenden lichtdurchlässigen Material gestreut wird, wird durch die Reflexionsschichten reflektiert, wobei eine Bündelung und Fokussierung erfolgt. Die V-Form der Nut ist nicht eng zu verstehen. Der Winkelbereich, in dem die beiden Reflexionsschichten zusammentreffen, kann abgerundet sein. Auch eine parabelförmige Nut ist als unter den Begriff "V-förmig" fallend anzusehen. Wichtig ist, dass die Nutflanken nach außen divergieren, so dass die Reflexionsschicht Streustrahlung bündelt und an einer Stelle außerhalb des Katheterquerschnitts fokussiert. Die Streustrahlung innerhalb des lichtdurchlässigen Materials kann dadurch entstehen, dass das lichtdurchlässige Material mit verteilt angeordneten dispergierenden Partikeln versehen wird.

Vorzugsweise ist der Lichtleiter in einem Mittelabschnitt des Katheters ebenfalls in einer Nut an der Außenseite des Katheters untergebracht. Insbesondere kann der Lichtleiter auf seiner gesamten Länge in einer Nut an der Außenseite des Katheters untergebracht sein. Dadurch wird vermieden, dass der Lichtleiter in ein Katheterlumen eingezogen werden muss. Vielmehr kann der Lichtleiter separat bearbeitet werden, bevor er in den Katheter eingesetzt wird. Das Einsetzen in den Katheter erfolgt von außen durch Einfügen in die nach außen offene Nut.

Gemäß einer bevorzugten Ausgestaltung der Erfindung besteht der Katheter in dem Mittelabschnitt und dem distalen Endabschnitt aus zwei separaten, an einer Katheterspleißstelle zusammengefügten Katheterkomponenten. Der Lichtleiter erstreckt sich einstückig über den Mittelabschnitt und den distalen Endabschnitt. Während der Katheter aus zwei Katheterkomponenten zusammengefügt ist, ist der Lichtleiter einstückig. Dadurch werden die Energieverluste minimiert.

Eine bevorzugte Ausführungsform der Erfindung sieht vor, dass der Katheter mindestens einen Kühlkanal aufweist, der in dem distalen Endabschnitt mit Austrittsbohrungen versehen ist. Dadurch kann Kühlflüssigkeit direkt an das wärmebehandelte Körpergewebe gebracht werden. Vorzugsweise sind zwei Kühlkanäle vorgesehen, die zu beiden Seiten des Lichtleiters angeordnet sind und deren Austrittsbohrungen zueinander gerichtet sind. Hierbei werden zwei Kühlflüsse an der Behandlungsstelle des Körpergewebes zusammengeführt, wodurch mit wenig Kühlmedium eine effektive lokal begrenzte Kühlung erreicht wird.

Zur Glättung der Außenseite des Katheters kann in dem distalen Endabschnitt ein lichtdurchlässiger Umschlauch und in dem Mittelabschnitt ein lichtundurchlässiger Umschlauch vorgesehen sein, wobei die beiden Umschläuche an einer Schlauchspleißstelle verbunden sind.

Im Folgenden wird unter Bezugnahme auf die Zeichnungen ein Ausführungsbeispiel der Erfindung näher erläutert.

Es zeigen:
- Figur 1: eine schematische Darstellung der generellen Struktur des Lichtleiters,
- Figur 2: einen Querschnitt entlang der Linie II-II im Mittelabschnitt des Katheters und
- Figur 3: einen Querschnitt entlang der Linie III-III im distalen Endabschnitt des Katheters.

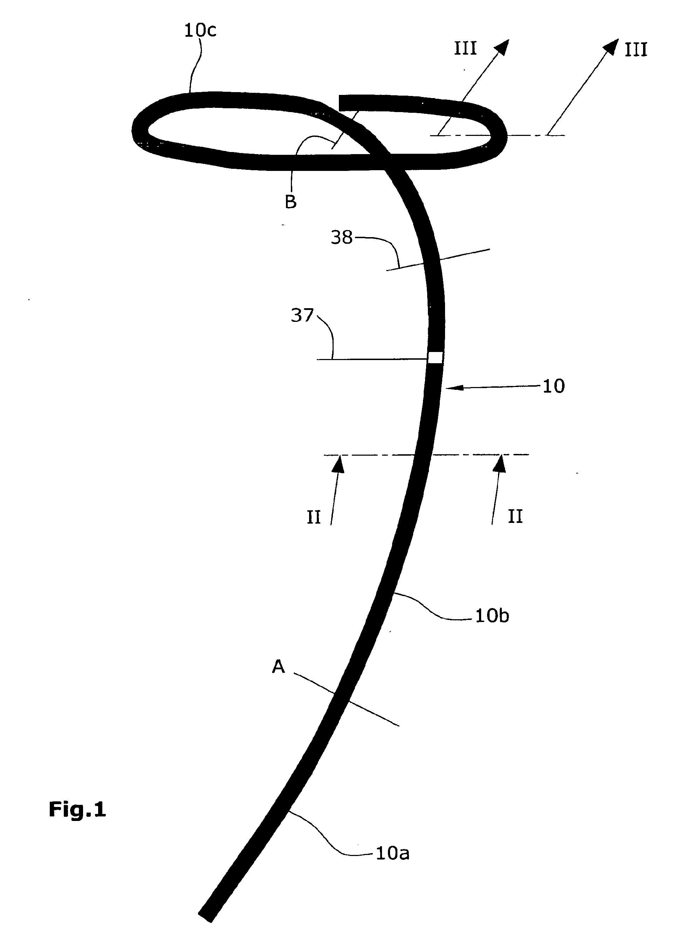

Der Laserapplikator weist einen Katheter 10 in Form eines langgestreckten Stranges auf. Der Katheter enthält eines oder mehrere Lumen. Er ist in der in Figur 1 dargestellten Weise vorgeformt und besteht aus einem proximalen Abschnitt 10a, einem Mittelabschnitt 10b und einem distalen Endabschnitt 10c. Während die Abschnitte 10a und 10b im wesentlichen geradlinigen Verlauf haben, ist der distale Endabschnitt 10c zu einer Schleife geformt, die einen an einer Stelle offenen Kreis bildet. Die Schleifenebene liegt quer, insbesondere rechtwinklig, zur Längsrichtung des Mittelabschnitt 10b. Sie hat eine solche Größe, dass sie sich von innen mit leichtem Druck gegen die Wand eines Blutgefäßes legt. Der Außendurchmesser der Schleife beträgt etwa 20 - 40 mm.

Die Stelle A bezeichnet den Übergang vom proximalen Abschnitt 10a zum Mittelabschnitt 10b. Die Stelle B bezeichnet den Übergang von dem Mittelabschnitt 10b in den distalen Endabschnitt 10c.

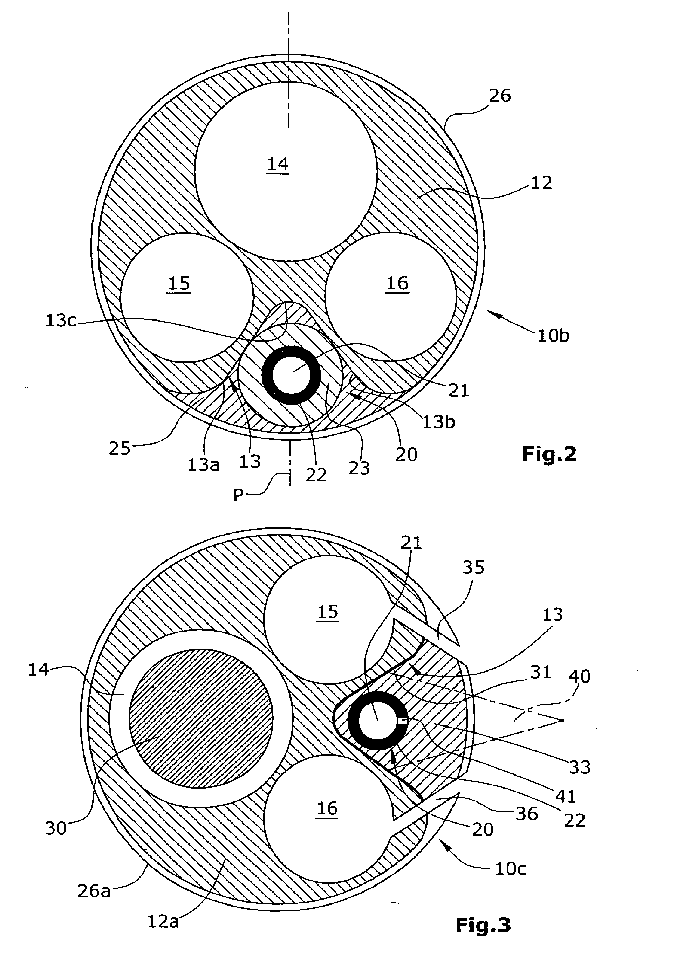

Figur 2 zeigt einen Querschnitt des Katheters in dem Mittelabschnitt 10b. Der Katheter weist einen einstückigen langgestreckten Katheterkörper 12 mit einem Durchmesser von 2 - 3 mm auf, der im wesentlichen kreisrunden Querschnitt hat und mit einer längslaufenden, im wesentlichen V-förmigen Nut 13 versehen ist. Die Nut 13 weist zwei nach außen divergierende Flanken 13a, 13b auf, die durch eine bogenförmige Basis 13c verbunden sind. Die Nut erstreckt sich bis in die Nähe der Längsmittelachse des Katheterkörpers 12.

Der Katheterkörper 12 enthält ein Lumen 14 für einen Formungsdraht. Das Lumen 14 ist der Nut 13 diametral gegenüberliegend angeordnet. Außerdem sind zwei längslaufende Kühlkanäle 15, 16 vorgesehen, die sich über die gesamte Katheterlänge erstrecken und symmetrisch zu der Längsmittelebene P angeordnet sind, die eine Symmetrieebene bildet und durch den Mittelpunkt des Lumens 14 und durch die Mittelebene der Nut 13 hindurchgeht. Der Katheterkörper 12 besteht aus einem Profilstrang von über die Länge einheitlichem Profil aus einem Elastomermaterial.

In die Nut 13 ist von außen her ein Lichtleiter 20 eingesetzt. Dieser besteht aus einem Kern 21 aus einer Glasfaser sowie einem den Kern 21 umgebenden Mantel 22 (cladding) aus einem Material mit höherem Brechungsindex als der Kern 21. Der Mantel 22 ist von einer Schutzhülle 23 umgeben, die einen Bruchschutz darstellt. Der gesamte Lichtleiter 20 hat einen solchen Durchmesser, dass er sich in die Nut 13 einfügt, ohne über die Kreiskontur des Katheters hinauszuragen.

Der Lichtleiter 20 ist in der Nut 13 mit einem Kleber 25 befestigt, welcher die gesamte Nut ausfüllt und eine Außenfläche hat, die der Kreiskontur des Katheterkörpers entspricht. Der Kleber 25 ist für die Strahlung undurchlässig. Der Katheter ist außen mit einem undurchsichtigen Umschlauch 26 umhüllt.

In dem distalen Endabschnitt 10c hat der Katheter den in Figur 3 dargestellten Querschnitt. Er weist einen Katheterkörper 12a auf, der das gleiche Profil hat wie der Katheterkörper 12 des Mittelabschnitts. In dem Lumen 14 befindet sich ein Formungsdraht 30, der dem distalen Endabschnitt 10c die in Figur 1 dargestellte Schleifenform gibt, jedoch elastisch ist und gestreckt werden kann. Von dem Lichtleiter 20 ist im distalen Endabschnitt 10c die Schutzhülle 23 entfernt. Die Nut 13 ist hier mit einer Reflexionsschicht 31 versehen, welche die Flanken der Nut und die Basis 13c bedeckt. Der Lichtleiter 20 weist in dem distalen Endabschnitt nur den Kern 21 und den Mantel 22 auf. Er ist in die Nut eingebettet, wobei die Nut 13 mit einem lichtdurchlässigen Material 33 gefüllt ist. Hierbei handelt es sich um einen Kleber, der lichtstreuende Partikel enthält.

Auch im distalen Endbereich ist der Katheter mit einem Umschlauch 26a versehen, der in diesem Bereich jedoch lichtdurchlässig ist.

Im distalen Endabschnitt sind die Kühlkanäle 15, 16 mit Austrittsbohrungen 35, 36 versehen, die zueinander konvergieren und Kühlstrahlen nach außen senden. Die Austrittsbohrungen 35, 36 verlaufen unter einem spitzen Winkel zueinander. Sie bewirken, dass die Kühlstrahlen auf das Zielgebiet der Wärmestrahlung auftreffen. Die Austrittsbohrungen 35, 36 haben entsprechende Öffnungen in dem Umschlauch.

In dem Auskoppelbereich 40 (Figur 1), in dem die Strahlung aus dem Lichtleiter 20 ausgekoppelt wird, ist der Mantel 22 des Lichtleiters mit Öffnungen 41 versehen, durch die die Strahlung aus dem Kern 21 ausgekoppelt wird. Der Auskoppelbereich 40 ist, bezogen auf die Schleife des distalen Endabschnitts, radial nach außen gerichtet. Die Symmetrieebene P (Figur 2) befindet sich in der Ebene der Schleife.

Der Kern 21 des Lichtleiters 20 und der Mantel 22 sind über die gesamte Länge des Katheters 10 durchgehend, so dass die Glasfaser des Lichtleiters nicht unterbrochen ist. Die Katheterkörper 12 und 12a sind an einer Katheterspleißstelle 37 miteinander verbunden. Die Umschläuche 26 und 26a sind an einer Schlauchspleißstelle 38 miteinander verbunden, die im Abstand von der Katheterspleißstelle 37 angeordnet ist, im vorliegenden Fall distal davon.

Bei der Herstellung des Laserapplikators werden zunächst die Katheterkörper 12, 12a aus dem gleichen Schlauchprofil geschnitten. Der Katheterkörper 12a wird anschließend mit der Reflexionsschicht 31 versehen. Hierbei handelt es sich um eine Metallschicht, die durch Sputtern oder Aufdampfen erzeugt wird.

Der Lichtleiter 20 wird zunächst außerhalb des Katheters bearbeitet, indem abschnittsweise die Schutzhülle 23 entfernt wird. In diesem Auskoppelbereich werden durch Laserbearbeitung die Öffnungen 41 in Form kleiner Bohrlöcher erzeugt. Der so vorbereitete Lichtleiter wird in die seitliche Nut 13 des Katheterkörpers 12 eingesetzt und anschließend mit dem Kleber 25 befestigt. Dann wird der Katheterkörper 12a passgenau mit dem Katheterkörper 12 an der Katheterspleißstelle 37 verbunden. Schließlich wird der Auskoppelbereich des Lichtleiters 20 in die seitliche Nut des Katheterkörpers 12 a eingesetzt und die Nut wird mit dem Material 33 ausgefüllt.

Schließlich werden die Umschläuche 26 und 26a auf die jeweiligen Katheterabschnitt aufgebracht.

Die Erfindung bietet den Vorteil, dass lediglich die Katheterkörper miteinander verspleißt werden und dass in gleicher Weise auch die Umschläuche verspleißt werden. Dagegen ist der Lichtleiter durchgehend, wobei er nur an dem Auskoppelbereich bearbeitet ist. Der Laserapplikator hat geringe Energieverluste und er ist imstande, eine hohe Energiekonzentration im Zielgebiet aufzubringen.

## Patentansprüche

1. Laserapplikator mit einem langgestreckten Katheter (10), der mindestens ein umfangsmäßig geschlossenes Lumen enthält, und einem längs des Katheters verlaufenden Lichtleiter (20), der in einem distalen Endabschnitt (10c) des Katheters (10) einen Auskoppelbereich (40) aufweist,
wobei der Auskoppelbereich (40) des Lichtleiters (20) in einer Nut (13) in der Außenseite des Katheters verläuft,
**dadurch gekennzeichnet,**
**dass** der Lichtleiter (20) in der Nut (13) mit einem die Nut füllenden, lichtdurchlässigen Material (33) fixiert ist, welches lichtstreuende Partikel enthält.

2. Laserapplikator nach Anspruch 1, **dadurch gekennzeichnet, dass** die Nut (13) V-förmig ist und Flanken (13a, 13b) aufweist, die mit einer Reflexionsschicht (31) versehen sind.

3. Laserapplikator nach Anspruch 1 oder 2, **dadurch gekennzeichnet, dass** der Lichtleiter (20) in einem Mittelabschnitt (10b) des Katheters (10) ebenfalls in einer Nut (13) an der Außenseite des Katheters verläuft.

4. Laserapplikator nach einem der Ansprüche 1 - 3, **dadurch gekennzeichnet, dass** der Katheter (10) in dem Mittelabschnitt (10b) und dem distalen Endabschnitt (10c) aus zwei separaten, an einer Katheterspleißstelle (37) zusammengefügten Katheterkomponenten besteht und dass der Lichtleiter (20) sich einstückig über den Mittelabschnitt (10b) und den distalen Endabschnitt (10c) erstreckt.

5. Laserapplikator nach Anspruch 4, **dadurch gekennzeichnet, dass** beide Katheterkomponenten gleiches Profil haben.

6. Laserapplikator nach einem der Ansprüche 1 - 5, **dadurch gekennzeichnet, dass** der Lichtleiter (20) einen lichtleitenden Kern (21) und einen den Kern umgebenden Mantel (22) aufweist, und mit Öffnungen (41) versehen ist.

7. Laserapplikator nach einem der Ansprüche 1 - 6, **dadurch gekennzeichnet, dass** der Lichtleiter (20) eine Schutzhülle (23) aufweist, die in dem Auskoppelbereich (40) entfernt ist.

8. Laserapplikator nach einem der Ansprüche 1 - 7, **dadurch gekennzeichnet, dass** der Katheter (10) mindestens einen Kühlkanal (15, 16) aufweist, der in dem distalen Endabschnitt (10c) mit Austrittsbohrungen (35, 36) versehen ist.

9. Laserapplikator nach Anspruch 8, **dadurch gekennzeichnet, dass** zwei Kühlkanäle (15, 16) vorgesehen sind, die zu beiden Seiten des Lichtleiters (20) angeordnet sind und deren Austrittsbohrungen zueinander gerichtet sind.

10. Laserapplikator nach Anspruch 9, **dadurch gekennzeichnet, dass** die Austrittsbohrungen (35, 36) beider Kühlkanäle (15, 16) in einem spitzen Winkel zueinander verlaufen.

11. Laserapplikator nach einem der Ansprüche 1 - 10, **dadurch gekennzeichnet, dass** der Katheter (10) mindestens in dem distalen Endabschnitt (10c) mit einem Umschlauch (26a) versehen ist.

12. Laserapplikator nach Anspruch 11, **dadurch gekennzeichnet, dass** in dem distalen Endabschnitt (10c) ein lichtdurchlässiger Umschlauch (26a) und in einem Mittelabschnitt (10b) ein lichtundurchlässiger Umschlauch (26) vorgesehen sind, wobei die beiden Umschläuche (26, 26a) an einer Schlauchspleißstelle (38) verbunden sind.

13. Laserapplikator nach einem der Ansprüche 1 - 12, **dadurch gekennzeichnet, dass** der Katheter (10) einen Formungsdraht (30) enthält, der den distalen Endabschnitt (10c) nach Art einer kreisförmigen Schleife rückstellbar formt.

14. Laserapplikator nach Anspruch 13, **dadurch gekennzeichnet, dass** der Formungsdraht (30) eine Drahtachse und der Lichtleiter (20) eine Lichtleiterachse aufweist und dass die Drahtachse und die Lichtleiterachse in einer Diametralebene (P) des Katheterquerschnitts liegen, wobei der Formungsdraht (30) in der Kreisförmige Schleife innen angeordnet ist.

## Claims

1. A laser applicator comprising an elongate catheter (10) including at least one circumferentially closed lumen, and light guide (20) extending along the catheter, said light guide having a decoupling portion (40) in a distal end section (10c) of the catheter (10),
wherein the decoupling portion (40) of the light guide (20) extends in a groove (13) in the outer side of the catheter,
**characterized in that**
the light guide (20) is fixed in the groove (13) by means of a translucent material (33) filling the groove, said material containing light-scattering particles.

2. The laser applicator of claim 1, **characterized in that** the groove (13) is V-shaped and has flanks (13a, 13b) provided with a reflective layer (31).

3. The laser applicator of claim 1 or 2, **characterized in that**, in a midsection (10b) of the catheter (10), the light guide (20) also extends in a groove (13) on the outer side of the catheter.

4. The laser applicator of one of claims 1 - 3, **characterized in that** the catheter (10) is formed by two separate catheter components, in the midsection (10b) and the distal end section (10c), joined at a catheter splice site (37) and that the light guide (20) extends integrally along the midsection (10b) and the distal end section (10c).

5. The laser applicator of claim 4, **characterized in that** both catheter components have the same profile.

6. The laser applicator of one of claims 1 - 5, **characterized in that** the light guide (20) comprises a light-guiding core (21) and a cladding (22) surrounding the core, and that it is provided with openings (41).

7. The laser applicator of one of claims 1 - 6, **characterized in that** the light guide (20) has a protective sheath (23) that is removed in the decoupling portion (40).

8. The laser applicator of one of claims 1 - 7, **characterized in that** catheter (10) comprises at least one cooling channel (15, 16) provided with outlet bores (35, 36) in the distal end section (10c).

9. The laser applicator of claim 8, **characterized in that** two cooling channels (15, 16) are provided that are arranged on both sides of the light guide (20) and whose outlet bores are directed towards each other.

10. The laser applicator 9, **characterized in that** the outlet bores (35, 36) of both cooling channels (15, 16) extend under an acute angle with respect to each other.

11. The laser applicator of one of claims 1 - 10, **characterized in that** the catheter (10) is provided with a covering hose (26a) at least in the distal end section (10c).

12. The laser applicator of claim 11, **characterized in that** a translucent covering hose (26a) is provided in the distal end section (10c) and an opaque covering hose (26) is provided in the midsection (10b), said two covering hoses (26, 26a) being joined at a hose splice site (38).

13. The laser applicator of one of claims 1 - 12, **characterized in that** the catheter (10) includes a forming wire (30) that resiliently forms the distal end section (10c) in the manner of a circular loop.

14. The laser applicator of claim 13, **characterized in that** the forming wire (30) has a wire axis and the light guide (20) has a light guide axis, and that the wire axis and the light guide axis lie in a diametrical plane (P) of the catheter cross section, the forming wire (30) being arranged on the inside in the circular loop.

## Revendications

1. Applicateur laser doté d'un cathéter de forme allongée (10) contenant au moins une lumière fermée circonférentiellement, et d'un câble fibre optique (20) s'étendant le long du cathéter et présentant, dans une portion d'extrémité distale (10c) du cathéter (10), une région de découplage (40),
la région de découplage (40) du câble fibre optique (20) s'étendant dans une gorge (13) pratiquée sur la face externe du cathéter,
**caractérisé en ce que**
le câble fibre optique (20) est fixé dans la gorge (13) au moyen d'un matériau (33) translucide remplissant la gorge, lequel contient des particules diffusant la lumière.

2. Applicateur laser selon la revendication 1, **caractérisé en ce que** la gorge (13) est en forme de V et présente des flancs (13a, 13b) qui sont pourvus d'une couche réflectrice (31).

3. Applicateur laser selon la revendication 1 ou 2, **caractérisé en ce que** le câble fibre optique (20), dans une portion médiane (10b) du cathéter (10), s'étend également dans une gorge (13) pratiquée sur la face externe du cathéter.

4. Applicateur laser selon l'une des revendications 1 à 3, **caractérisé en ce que**, dans la portion médiane (10b) et la portion d'extrémité distale (10c), le cathéter (10) est constitué de deux composants de cathéter séparés, assemblés en un point d'épissure (37), et **en ce que** le câble fibre optique (20) s'étend d'un seul tenant sur la portion médiane (10b) et la portion d'extrémité distale (10c).

5. Applicateur laser selon la revendication 4, **caractérisé en ce que** les deux composants de cathéter ont un profil identique.

6. Applicateur laser selon l'une des revendications 1 à 5, **caractérisé en ce que** le câble fibre optique (20) présente une âme (21) propageant la lumière et une enveloppe (22) entourant l'âme, et est pourvu d'ouvertures (41).

7. Applicateur laser selon l'une des revendications 1 à 6, **caractérisé en ce que** le câble fibre optique (20) présente une gaine de protection (23) qui est éliminée dans la région de découplage (40).

8. Applicateur laser selon l'une des revendications 1 à 7, **caractérisé en ce que** le cathéter (10) présente au moins un canal de refroidissement (15, 16) qui est pourvu d'orifices de sortie (35, 36) dans la portion d'extrémité distale (10c).

9. Applicateur laser selon la revendication 8, **caractérisé en ce que** sont prévus deux canaux de refroidissement (15, 16) disposés des deux côtés du câble fibre optique (20) et dont les orifices de sortie sont orientés l'un vers l'autre.

10. Applicateur laser selon la revendication 9, **caractérisé en ce que** les orifices de sortie (35, 36) des deux canaux de refroidissement (15, 16) forment entre eux un angle aigu.

11. Applicateur laser selon l'une des revendications 1 à 10, **caractérisé en ce que** le cathéter (10) est pourvu, au moins dans la portion d'extrémité distale (10c), d'une gaine souple (26a).

12. Applicateur laser selon la revendication 11, **caractérisé en ce que** sont prévus dans la portion d'extrémité distale (10c) une gaine souple translucide (26a) et dans une portion médiane (10b) une gaine souple (26) opaque, les deux gaines souples (26, 26a) étant reliées en un point d'épissure (38).

13. Applicateur laser selon l'une des revendications 1 à 12, **caractérisé en ce que** le cathéter (10) contient un fil de formage (30) qui façonne avec reprise de forme la portion d'extrémité distale (10c) à la manière d'une boucle de forme circulaire.

14. Applicateur laser selon la revendication 13, **caractérisé en ce que** le fil de formage (30) présente un axe de fil et le câble fibre optique (20) un axe de câble fibre optique, et **en ce que** l'axe de fil et l'axe de câble fibre optique se situent dans un plan diamétral (P) de la section transversale du cathéter, le fil de formage (30) dans la boucle circulaire étant disposé à l'intérieur.
